(19) Europäisches Patentamt
European Patent Office
Office européen des brevets

(11) Publication number: **0 321 287**
**A2**

# (12) EUROPEAN PATENT APPLICATION

(21) Application number: **88311950.5**

(22) Date of filing: **16.12.88**

(51) Int. Cl.[4]: **A 61 K 31/13**
A 61 K 31/70

(30) Priority: **18.12.87 US 135073**

(43) Date of publication of application:
**21.06.89 Bulletin 89/25**

(84) Designated Contracting States: **DE FR GB**

(71) Applicant: **City of Hope**
**1500 East Duarte Road**
**Duarte California 91010 (US)**

(72) Inventor: **DeMeyts, Pierre**
**2473 Oswego Street, 5**
**Pasadena California, 91107 (US)**

**Smal, Jean**
**217 South Catalina Avenue, 3**
**Pasadena California 91106 (US)**

(74) Representative: **Hallybone, Huw George et al**
**CARPMAELS AND RANSFORD 43 Bloomsbury Square**
**London WC1A 2RA (GB)**

(54) **Inhibition of lipogenesis.**

(57) This invention pertains to the treatment and prevention of obesity in mammals, including humans, by the administration of a sphingolipid, preferably a lysosphingolipid or a therapeutically effective derivative thereof.

EP 0 321 287 A2

## Description

## INHIBITION OF LIPOGENESIS

This invention relates to the inhibition and the complete suppression of lipogenesis. More particularly, the invention relates to the treatment and prevention of obesity in mammals, including humans, by the administration of a sphingolipid, preferably a lysosphingolipid or a therapeutically effective derivative thereof.

### DESCRIPTION OF THE INVENTION

The excessive accumulation of lipids in fat cells is one of the major features of human and animal obesity. Pursuant to this invention a member of a family of complex lipids known to the art as sphingolipids and (lyso)sphingolipids is administered to mammals, including humans, to block or to completely suppress fat cell lipogenesis, and thus to treat or prevent mammal obesity.

Sphingolipids comprise a large group of long chain bases with a common 2 amino-1,3 diol system and constitute one of two main classes of complex lipids that participate in the lipid phase and surface structure of biological membranes. The sphingolipids include subgroups of divergent molecular structure such as ceramide, sphingomyelin, galacto-and glucocerebrosides, sulfatides, the neutral glycosphingolipids, the acidic gangliosides and the (lyso)sphingolipids in which the 2-amino group is unsubstituted.

Formula I includes some of the lysosphingolipids useful in the invention:

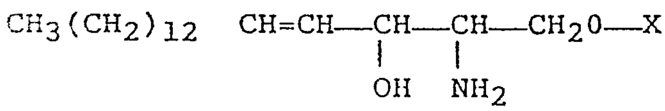

wherein X may be, e.g., H (sphingosine) galactose (psychosine) sulfogalactose,

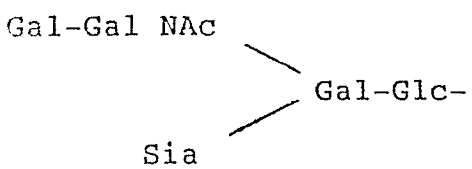
(lyso $GM_1$)

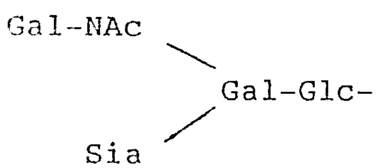
(lyso $GM_2$)

Sia-Gal-Glc- (lyso $GM_3$)

and derivatives thereof.

The preferred compound for use in this invention is sphingosine.

### EXEMPLIFICATION OF THE INVENTION

This invention involves the discovery that sphingolipids and (lyso)sphingolipids inhibit or completely suppress lipogenesis in the fat cells of mammals. The degree of inhibition is a function of the effective sphingolipid or (lyso)sphingolipid in or available to the fat cells. More particularly, it has been discovered that the stimulation of fat cell lipogenesis by insulin, growth hormone and phorbol esters is blocked reversibly by the action of a lipid useful in this invention at an effective but relatively lower concentration, whereas at higher concentrations basal lipogenesis (lipogenesis in the absence of hormones) is completely suppressed.

Figure 1 illustrates the effect of sphingosine on the maximal stimulation of lipogenesis in rat adipocytes by phorbol ester (PMA), human growth hormone (hGH) and insulin (INS).

The incorporation of the tracer D-[3]$^3$H]-glucose into lipids is expressed in counts per minute of radioactivity (= cpm) per tube. It is the mean of a triplicate measurement, plus or minus one standard deviation (1 S.D.). The values obtained have been divided by 1,000 for simplifying the graph (= X $10^{-3}$). Thus, a value of 3 on the

Figure I graph means that 3,000 cpm were actually measured.

Cells were incubated without hormone (basal-black bars) or with respectively 1000 ng/ml PMA, 1000 ng/ml hGH or 100 ng/ml insulin in the absence (bars c) or in the presence (bars s) of 50 μM of sphingosine (combined black and white bars).

At 100 μM sphingosine both basal and hormone stimulated lipogenesis are reduced to zero.

The lipogenesis assay has been conducted according to Smal, et al. J. Biol. Chem. 262:11071-11079 (1987). Briefly, dissected epididymal and retroperitoneal fat pads were digested under vigorous shaking at 37° C for 30 min with collagenase (1.0 mg/ml) in Krebs-Ringer-Hepes (KRH) buffer, pH 7.4, 35 mg/ml dialyzed bovine serum albumine (BSA), 0.27 mM glucose. After filtration on cheesecloth and 4 washes in KRH with 10 mg/ml BSA, the adipocytes were preincubated for 4 hours at 37° C in the same buffer. Sphingosine (100 mM stock solution in ethanol) was added to the cells (final concentration:50 μM) 10 min before the lipogenesis assay. The same concentration of ethanol, without sphingosine, was added to the control cells.

The lipogenesis assay was performed in triplicate in 6 ml polyethylene vials by adding successively 400 μl of adipocyte suspension (80 X $10^3$ cells/ml), 50 μl of KRH buffer pH 7.4 (1% BSA, 0.27 mM glucose) without hormone (basal lipogenesis) or with human growth hormone (1000 ng/ml), insulin (100 ng/ml) or phorbol ester PMA (1000 ng/ml) and 50 μl D-[3-$^3$H]-glucose in a total volume of 0.5 ml. The vials were incubated 2 hours at 37° C under gentle shaking. The incubation was interrupted by adding 5 ml/tube of toluene scintillator (1 liter toluene + 0.3 g of 1,4bis[2-(4-methyl-5phenyloxazolyl)benzene and 5 g of 2,5-diphenyloxazole under vigorous shaking (30s to break the cells) followed by a rest of at least 1 hour to allow extraction of lipids into the toluene phase before counting. The samples were counted in a Beckman LS 1880 beta counter. The counting efficiencies for the different samples were measured by internal standardization with quenched tritiated standards. The incorporation of D-[3-$^3$H]-glucose into lipids is expressed in cpm X $10^{-3}$/tube ± 1 standard deviation.

## Administration and Dosage

The selection of a mode of administration and of dosage level is within the competence and discretion of persons skilled in the art. The invention in its broader aspects includes a pharmaceutical composition containing a sphingolipid or a lysosphingolipid useful to block lipid metabolism in mammals, including man. The sphingolipid or lysosphingolipid may be administered per se or in combination with therapeutically acceptable adjuvants, e.g., liposomes.

The preferred (lyso)sphingolipid is sphingosine.

Parenteral administration is preferred. Percutaneous administration using gels or creams containing a permeabilizing agent such as dimethylsulfoxide may be used. The sphingolipid or lysosphingolipid is administered in an amount therapeutically effective to partially or completely suppress the hormonal or basal stimulation of lipogenesis in mammalian fat cells.

The degree of suppression is a function of dosage level. Administration of at least about 15 mg/kg is appropriate to partially suppress lipogenesis. Administration of at least about 60 mg/kg is indicated for the complete suppression of basal lipogenesis. Administration may appropriately continue over an appropriate time period until the desired therapeutic effect is observed.

A number of drugs are known to interfere with lipogenesis e.g., hormone (catecholamines, glucagon, ACTH) clofibrate, halogenate, fenfluramine, amphetamine, cinchocaine, chlorpromazine, and some derivatives thereof. None of these drugs suppress lipogenesis as effectively as the lipids of this invention.

## Claims

1. A sphingolipid or a (lyso)sphingolipid for use as a pharmaceutical.
2. A sphingolipid or a (lyso)sphingolipid for use in the treatment of obesity in a mammal.
3. Sphingosine for use as a pharmaceutical.
4. Sphingosine for use in the treatment of obesity in a mammal.
5. Use of a sphingolipid or a (lyso)sphingolipid in the manufacture of a medicament for the treatment of obesity in a mammal.
6. Use of a sphingolipid or a (lyso)sphingolipid for the manufacture of a medicament to suppress basal lipogenesis in a mammal.
7. Use of a sphingolipid or a (lyso)sphingolipid for the manufacture of a medicament to block basal fat accumulation in a mammal.
8. Use of a (lyso)sphingolipid for the manufacture of a medicament to block insulin-stimulated fat accumulation in a mammal.
9. Use according to any one of claims 5 to 8 wherein the mammal is a human.
10. Use according to any one of claims 5 to 9 wherein the (lyso)sphingolipid is sphingosine.

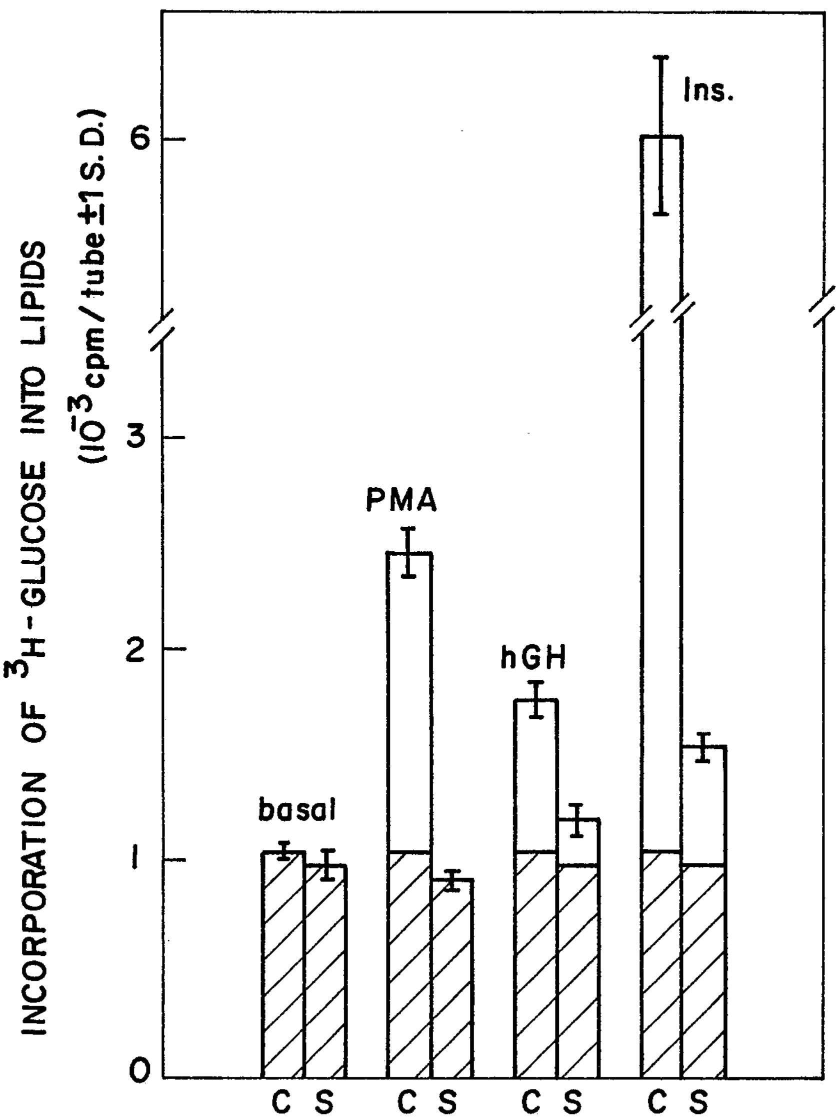
INCORPORATION OF $^3$H-GLUCOSE INTO LIPIDS
($10^{-3}$ cpm/tube ±1 S.D.)
0
1
2
3
6
basal
PMA
hGH
Ins.
C S
C S
C S
C S

FIG. I